# EUROPÄISCHE PATENTSCHRIFT

(11) **EP 2 709 576 B1**
(45) Veröffentlichungstag und Bekanntmachung des Hinweises auf die Patenterteilung: **11.04.2018**
(21) Anmeldenummer: 11720390.1
(22) Anmeldetag: 16.05.2011
(51) Int. Cl.: A61F 9/008

(54) **VERFAHREN ZUR KALIBRIERUNG EINES SYSTEMS ZUR CHIRURGISCHEN BEHANDLUNG EINES AUGES**
METHOD FOR CALIBRATING SYSTEM FOR SURGICAL TREATMENT OF AN EYE, AND
PROCÉDÉ POUR L'ÉTALONNAGE D'UN SYSTÈME POUR LE TRAITEMENT CHIRURGICAL D'UN OEIL.

(43) Veröffentlichungstag der Anmeldung: 26.03.2014
(73) Patentinhaber: WaveLight GmbH, 91058 Erlangen (DE)
(72) Erfinder: WELLHOEFER, Armin, 90571 Schwaig (DE); DONITZKY, Christof, 90542 Eckental/Eschenau (DE)
(74) Vertreter: Schicker, Silvia
(86) Internationale Anmeldenummer: PCT/EP2011/002415
(87) Internationale Veröffentlichungsnummer: WO 2012/155930

(56) Entgegenhaltungen:
- EP-A1- 1 279 385
- WO-A1-02/102262
- WO-A1-2010/102246
- WO-A2-2006/087180
- US-A1- 2007 173 792

## Beschreibung

Die Erfindung betrifft ein System und ein Verfahren zur chirurgischen Behandlung eines Auges, insbesondere unter Verwendung eines Lasers mit einer hohen Repetitionsrate. Ferner betrifft die Erfindung ein Verfahren zur Kalibrierung eines derartigen Systems zur chirurgischen Behandlung eines Auges.

In der refraktiven Augenchirurgie zur Behandlung von Fehlsichtigkeiten durch Photoablation der Kornea werden derzeit üblicherweise Argonfluorid-Excimerlaser eingesetzt, die ultraviolettes Licht mit einer Wellenlänge von 193 nm emittieren. Da die Kornea in diesem Wellenlängenbereich eine hohe Absorption aufweist, kann Korneagewebe bei minimaler Beanspruchung angrenzender Gewebeschichten präzise abgetragen werden. Die Abtragstiefe, das heißt die Dicke der durch den Laser abgetragenen Korneagewebeschicht, wird dabei durch entsprechende Steuerung der Pulsenergie gesteuert, wobei die Überwachung der Pulsenergie so erfolgen muss, dass Beeinträchtigungen des Patienten durch Veränderungen des an das abzutragende Korneagewebe angrenzenden Gewebes zuverlässig vermieden werden.

Gegenwärtig verwendete Excimerlasersysteme arbeiten mit Repetitionsraten von 200 Hz bis 750 Hz. Eine Erhöhung der Laserfrequenz würde jedoch eine Verkürzung der Behandlungsdauer ermöglichen, wodurch Komplikationen und die Dehydration der Kornea während der Operation minimiert werden könnten. Infolge dessen gibt es Bestrebungen, in der refraktiven Augenchirurgie Excimerlasersysteme mit einer erhöhten Repetitionsrate von bis zu 1050 Hz zum Einsatz zu bringen. Der erhöhten Frequenz der Lasersysteme muss jedoch bei der Steuerung der Pulsenergie Rechnung getragen werden, da andernfalls Schäden an dem behandelten Auge nicht sicher auszuschließen wären.

Die Druckschrift WO 2006/087180 A2 offenbart ein Verfahren zur Erstellung eines Ablationsprogramms für eine Ablation von Material von einer Oberfläche eines Körpers gemäß einem vorgegebenen Soll-Ablationsprofil durch die Abgabe von Pulsen eines gepulsten Laserstrahls auf die Oberfläche, bei dem das Ablationsprogramm aus dem Soll-Ablationsprofil in Abhängigkeit von der Form eines Strahlprofils des Laserstrahls und von einer Neigung der zu ablatierenden

Oberfläche und/oder unter Berücksichtigung eines Wassergehalts des zu ablatierenden Materials erstellt wird.

Die Druckschrift WO 02/102262 A1 offenbart ein Verfahren und eine zugehörige Anordnung zum Abtragen von Material mit Hilfe von Laserstrahlung. Dabei wird die Temperatur und/oder die Feuchtigkeit am Abtragungsort mittels eines in vorgegebener Richtung über den Abtragungsort hinweg strömenden Gases im Wesentlichen konstant gehalten. Das Gas hat dabei während des Abtragungsprozesses konstante oder variierende Temperaturen, Feuchtigkeitswerte und Strömungsgeschwindigkeiten. Eine Anordnung zur Ausübung des Verfahrens weist einen röhrenförmigen Kanal auf, durch dessen Ende hindurch ein Laserstrahl auf eine die Oberfläche eines Gegenstandes trifft und dort Material abträgt. Dabei ist aus Austrittsöffnungen eines Strömungskanals, der über einen Anschlussstutzen mit einer Fördereinrichtung verbunden ist, eine temperierte Luftströmung mit definierter Feuchte auf den Abtragungsort gerichtet.

Die Druckschrift US 2007/173792 A1 offenbart ein Verfahren zum Testen eines Augenlaserchirurgiesystems, umfassend: Abbilden eines bekannten Kalibrierungsmusters an einem Bildort mit einer Abbildungsvorrichtung; Herstellen einer Bildskala basierend auf dem Kalibrierungsmuster und dem Bild; bildgemäßes Verändern einer Reihe von Bereichen von einer Testoberfläche mit einem Laserstrahl von dem System an dem Abbildungsort; seitliches Umlenken des Laserstrahls gemäß einem beabsichtigten Muster zwischen Verändern der Bereiche unter Verwendung eines Strahlabgabesystems, um ein Testmuster auf der Testoberfläche zu bilden; Abbilden des Testmusters an dem Abbildungsort mit der Abbildungsvorrichtung; Bestimmen einer seitlichen Umlenkungscharakteristik von dem Strahlabgabesystem basierend auf der Bildskala, dem beabsichtigten Muster und dem Testmusterbild; und Qualifizieren oder Kalibrieren des Strahlabgabesystems in Reaktion auf die seitliche Umlenkungscharakteristik.

Die Druckschrift WO 2010/102246 A1 offenbart ein laserchirurgisches Verfahren, mit den Schritten - Bereitstellen einer laserchirurgischen Vorrichtung mit einer Handvorrichtung, die eine optische Zuführkomponente zum Senden von Laserenergie aus einer Quelle zu einem Zielvolumen und einen Beschleunigungssensor zur Positionsermittlung der Handvorrichtung umfasst; - Verwenden der Handvorrichtung zum Senden der Laserenergie aus der Quelle zu einer Vielzahl von Positionen innerhalb des Zielvolumens; - Verwenden des Beschleunigungssensors zur Positionsermittlung der Handvorrichtung; - Bestimmen eines Energiebetrags, der jeder der Vielzahl von Positionen innerhalb des Zielvolumens zugeführt wird, auf der Grundlage der ermittelten Position; und - Anzeigen einer grafischen Darstellung des Energiebetrags.

Die Druckschrift EP 1 279 385 A1 offenbart eine Technik, bei der während der jeweiligen Bestrahlungszeit der Behandlungsstrahlung zusätzliche Strahlungspulse mit geringerer Pulsdauer und geringerer Energie als bei der Behandlungsstrahlung auf das behandelte biologische Gewebe gerichtet werden oder die Behandlungsstrahlung kurzzeitig abgeschaltet und wieder eingeschaltet wird. Die zusätzlichen Strahlungspulse oder die kurzzeitigen Abschaltungen der Behandlungsstrahlung können im Wesentlichen in gleichen Zeitabständen erfolgen. Die bei Anwendung der zusätzlichen Strahlungsimpulse entstehenden thermischen Gewebeexpansionen und die bei den kurzzeitigen Abschaltungen der Behandlungsstrahlung entstehenden Gewebekontraktionen werden durch Druckmessung oder durch optische Messung erfasst. Aus den jeweiligen Messsignalen, welche von den zusätzlichen Strahlungspulsen (Messstrahlungspulsen) oder den kurzzeitigen Abschaltungen der Behandlungsstrahlung veranlasst werden, wird die Temperaturerhöhung bestimmt, insbesondere werden die jeweiligen Absolutwerte der Temperatur bestimmt.

Der Erfindung liegt die Aufgabe zugrunde, ein Verfahren zur Kalibrierung eines Systems zur chirurgischen Behandlung eines Auges anzugeben, das es ermöglichen, unerwünschte Veränderungen von Gewebeschichten, die an mittels eines Lasers abzutragende Gewebeschichten angrenzen, zu vermeiden.

Diese Aufgabe wird durch ein Verfahren zur Kalibrierung eines Systems zur chirurgischen Behandlung eines Auges gemäß dem unabhängigen Anspruch gelöst. Entwicklungen sind in den abhängigen Ansprüchen dargelegt.

Ein System zur chirurgischen Behandlung eines Auges umfasst eine Laseranordnung, die dazu eingerichtet ist, Licht mit einer zur chirurgischen Behandlung des Auges geeigneten Wellenlänge und Repetitionsrate zu emittieren. Die Laseranordnung kann beispielsweise einen Excimerlaser, insbesondere einen Argonfluorid-Excimerlaser umfassen, der ultraviolettes Licht mit einer Wellenlänge von 193 nm emittiert. Grundsätzlich kann es sich bei der Laseranordnung um eine herkömmliche Laseranordnung handeln, die mit einer Repetitionsrate von 200 Hz bis 750 Hz arbeitet. Vorzugsweise ist die Laseranordnung jedoch dazu in der Lage, eine erhöhte Repetitionsrate von beispielsweise 1050 Hz oder mehr zu verwirklichen.

Ferner umfasst das Behandlungssystem eine Temperaturerfassungseinrichtung, die dazu eingerichtet ist, die Temperatur eines mit dem Licht der Laseranordnung zu beaufschlagenden Objekts zu erfassen. Die Temperaturerfassungseinrichtung kann eine beliebige Messeinrichtung sein, die dazu in der Lage ist, die Temperatur des mit dem Licht der Laseranordnung zu beaufschlagenden Objekts zu erfassen. Das Objekt ist ein Testobjekt aus einem Kunststoffmaterial, das zur Kalibrierung des Systems zur chirurgischen Behandlung eines Auges, wie später noch näher erläutert werden wird, mit dem Licht der Laseranordnung beaufschlagt werden kann. Die mittels der Temperaturerfassungseinrichtung zu erfassende Temperatur kann die Temperatur eines mit dem Licht der Laseranordnung zu beaufschlagenden Testobjekts aus einem Kunststoffmaterial vor der Beaufschlagung mit dem Licht der Laseranordnung oder die Temperatur eines mit dem Licht der Laseranordnung zu beaufschlagenden Testobjekts aus einem Kunststoffmaterial während der Beaufschlagung mit dem Licht der Laseranordnung sein. Ein Behandlungssystem, das nicht Teil der Erfindung ist, ermöglicht es in vorteilhafter Weise, die Temperatur eines von einer Laseranordnung mit Licht zu beaufschlagenden Objekts bei der Beaufschlagung des Objekts mit dem Licht der Laseranordnung zu berücksichtigen. Beispielsweise kann mit Hilfe eines solchen erfindungsgemäßen Systems die Ausgangstemperatur eines zu behandelnden Auges oder eines Testobjekts, d. h. die Temperatur, die das Auge oder das Testobjekt vor der Behandlung mit dem von der Laseranordnung emittierten Licht aufweist, bei der Behandlung des Auges oder des Testobjekts mit dem Licht der Laseranordnung berücksichtigt werden. Alternativ oder zusätzlich dazu ermöglicht es das Behandiungssystem, die Auswirkung zu erfassen, die das Licht der Laseranordnung auf die Temperatur eines mit dem Licht der Laseranordnung beaufschlagten Objekts hat. Insbesondere kann mittels der Temperaturerfassungseinrichtung eine durch die Beaufschlagung mit dem Licht der Laseranordnung verursachte Temperaturerhöhung des Objekts erfasst werden.

Dadurch können beispielsweise durch zu hohe Temperaturen verursachte Schäden an einem mittels des Behandiungssystems behandelten Auge minimiert oder vermieden werden. Das bei der chirurgischen Behandlung eines Auges vorhandene Komplikationsrisiko kann somit verringert werden. Die Ausstattung eines Systems zur chirurgischen Behandlung eines Auges mit einer Temperaturerfassungseinrichtung ist insbesondere dann sinnvoll, wenn das System eine Laseranordnung umfasst, die dazu in der Lage ist, Licht mit einer hohen Repetitionsrate von beispielsweise 1050 Hz zu emittieren, da eine hohe Repetitionsrate der Laseranordnung eine verstärkte Temperaturerhöhung in einem mit dem Licht der Laseranordnung beaufschlagten Objekt zur Folge hat. Die Temperaturerfassungseinrichtung kann jedoch auch in einem mit einer herkömmlichen Laseranordnung mit einer Repetitionsrate von beispielsweise 200 Hz bis 750 Hz ausgestatteten Behandlungssystem in vorteilhafter Weise zur Optimierung des Behandlungsprozesses unter Vermeidung von Temperaturschäden an dem zu behandelnden Auge eingesetzt werden.

Die Temperaturerfassungseinrichtung des Behandlungssystems, das nicht Teil der Erfindung ist, kann dazu eingerichtet sein, die Temperatur des mit dem Licht der Laseranordnung zu beaufschlagenden Objekts vor der Beaufschlagung des Objekts mit dem Licht der Laseranordnung zu erfassen. Mit anderen Worten, die Temperaturerfassungseinrichtung kann dazu eingerichtet sein, eine Ausgangstemperatur des mit dem Licht der Laseranordnung zu beaufschlagenden Objekts zu messen. Zusätzlich oder alternativ dazu kann die Temperaturerfassungseinrichtung jedoch auch dazu eingerichtet sein, die Temperatur des mit dem Licht der Laseranordnung zu beaufschlagenden Objekts während der Beaufschlagung des Objekts mit dem Licht der Laseranordnung zu erfassen. Insbesondere kann die Temperaturerfassungseinrichtung dazu eingerichtet sein, eine kontinuierliche Erfassung der Temperatur des mit dem Licht der Laseranordnung zu beaufschlagenden Objekts während der Beaufschlagung des Objekts mit dem Licht der Laseranordnung zu realisieren. Eine Temperaturerfassungseinrichtung kann einen vorzugsweise berührungslos arbeitenden Sensor zur Erfassung der Temperatur eines mit dem Licht der Laseranordnung zu beaufschlagenden Objekts umfassen. Alternativ dazu ist auch eine Ausstattung einer Temperaturerfassungseinrichtung mit einer Einrichtung zur Erfassung von UV-Rückstrahlung von dem mit dem Licht der Laseranordnung zu beaufschlagenden Objekt denkbar, anhand derer eine Abschätzung der Temperatur des mit dem Licht der Laseranordnung zu beaufschlagenden Objekts möglich ist. Vorzugsweise ist eine Temperaturerfassungseinrichtung jedoch mit einer Wärmebildkamera ausgestattet.

Eine Wärmebildkamera ist insbesondere dann vorteilhaft, wenn die Temperaturerfassungseinrichtung dazu eingesetzt werden soll, die Temperatur des mit dem Licht der Laseranordnung zu beaufschlagenden Objekts während der Beaufschlagung des Objekts mit dem Licht der Laseranordnung zu erfassen, da die Wärmebildkamera die Beaufschlagung des Objekts mit dem Licht der Laseranordnung nicht beeinträchtigt, jedoch problemlos eine Temperaturerfassung in Echtzeit ermöglicht. Die von dem mit dem Licht der Laseranordnung zu beaufschlagenden Objekt abgegebene IR-Strahlung kann der Wärmebildkamera direkt zugeleitet werden. Falls gewünscht oder erforderlich, kann jedoch auch mindestens eine Umlenkeinrichtung, beispielsweise ein Umlenkspiegel oder dergleichen, im Strahlengang der von dem mit dem Licht der Laseranordnung zu beaufschlagenden Objekt abgegebenen IR-Strahlung angeordnet werden. Ein System zur chirurgischen Behandlung eines Auges, das nicht Teil der Erfindung ist, umfasst vorzugsweise ferner eine Steuereinrichtung, die dazu eingerichtet ist, ein von der Temperaturerfassungseinrichtung ausgegebenes Temperatursignal zu empfangen und die Laseranordnung in Abhängigkeit dieses Temperatursignals zu steuern. Beispielsweise kann die Steuereinrichtung dazu eingerichtet sein, ein Temperatursignal zu empfangen, das für eine gemittelte Temperatur über eine definierte Fläche des mit dem Licht der Laseranordnung zu beaufschlagenden Objekts, die beispielsweise 20x20 mm groß sein kann, charakteristisch ist. Die Steuereinrichtung kann dazu eingerichtet sein, die Laseranordnung in Abhängigkeit einer vor der Beaufschlagung des Objekts mit dem Licht der Laseranordnung gemessenen Ausgangstemperatur des mit dem Licht der Laseranordnung zu beaufschlagenden Objekts zu steuern. Beispielsweise kann die Steuereinrichtung dazu eingerichtet sein, die Pulsenergie der Laseranordnung an die gemessene Ausgangstemperatur des mit dem Licht der Laseranordnung zu beaufschlagenden Objekts anzupassen. Alternativ oder zusätzlich dazu kann die Steuereinrichtung jedoch auch dazu eingerichtet sein, die Laseranordnung während der Beaufschlagung des Objekts mit dem Licht der Laseranordnung in Abhängigkeit des von der Temperaturerfassungseinrichtung ausgegebenen Temperatursignals zu steuern.

Insbesondere kann die Steuereinrichtung dazu eingerichtet sein, die Pulsenergie der Laseranordnung zu reduzieren oder einen Ablationsbereich zu einem späteren Zeitpunkt nochmals anzusteuern, wenn die von der Temperaturerfassungseinrichtung erfasste Temperatur des mit dem Licht der Laseranordnung beaufschlagten Objekts einen vorbestimmten Grenzwert überschreitet. Falls gewünscht oder erforderlich, kann die Steuereinrichtung auch dazu eingerichtet sein, die Laseranordnung abzuschalten, wenn die von der Temperaturerfassungseinrichtung erfasste Temperatur des mit dem Licht der Laseranordnung beaufschlagten Objekts einen vorbestimmten Grenzwert überschreitet. Durch eine derartige Ausgestaltung eines Behandlungssystems können durch eine unerwünschte Temperaturerhöhung verursachte Schäden an einem zu behandelnden Auge verringert bzw. vollständig vermieden werden. Dadurch können Patienten beispielsweise vor Augenschäden geschützt werden, die durch den Ausfall eines Scanners oder beider Scanner verursacht werden, der/die den Laserstrahl über die Oberfläche des zu behandelnden Auges führt/führen.

Bei einem Verfahren zur chirurgischen Behandlung eines Auges wird das Auge mit Licht, das von einer Laseranordnung mit einer zur chirurgischen Behandlung des Auges geeigneten Wellenlänge und Repetitionsrate emittiert wird, beaufschlagt. Das von der Laseranordnung emittierte Licht kann beispielsweise ultraviolettes Licht mit einer Wellenlänge von 193 nm sein. Die Repetitionsrate der Laseranordnung kann 200 Hz bis 750 Hz betragen, aber auch deutlich höher sein und beispielsweise bei 1050 Hz oder mehr liegen. Ferner wird bei einem Verfahren zur chirurgischen Behandlung eines Auges die Temperatur des mit dem Licht der Laseranordnung zu beaufschlagenden Auges erfasst.

Die Temperatur des mit dem Licht der Laseranordnung zu beaufschlagenden Auges kann mittels der Temperaturerfassungseinrichtung erfasst werden, bevor und/oder während das Auge mit dem Licht der Laseranordnung beaufschlagt wird. Wenn die Temperaturerfassung vor der Beaufschlagung des Auges mit dem Licht der Laseranordnung erfolgt, d. h. die Ausgangstemperatur des Auges bestimmt wird, können, falls erforderlich, Maßnahmen zur Anpassung der Temperatur des mit dem Licht der Laseranordnung zu beaufschlagenden Auges an eine Soll-Temperatur ergriffen werden. Beispielsweise kann das Auge, insbesondere die Kornea des Auges, gekühlt werden, um dessen/deren Temperatur vor der Beaufschlagung mit dem Licht der Laseranordnung zu reduzieren.

Die Temperatur des mit dem Licht der Laseranordnung zu beaufschlagenden Auges wird vorzugsweise berührungslos mit Hilfe einer Wärmebildkamera erfasst.

Bei einem Verfahren zur chirurgischen Behandlung eines Auges wird die Laseranordnung in Abhängigkeit eines von der Temperaturerfassungseinrichtung ausgegebenen Temperatursignals gesteuert. Beispielsweise kann die Steuerung der Laseranordnung an eine gemessene Ausgangstemperatur des mit dem Licht der Laseranordnung zu beaufschlagenden Auges angepasst werden. Zusätzlich oder alternativ dazu kann die Laseranordnung während der chirurgischen Behandlung des Auges, d. h. während das Auge mit dem Licht der Laseranordnung beaufschlagt wird, in Abhängigkeit eines von der Temperaturerfassungseinrichtung ausgegebenen Temperatursignals gesteuert werden.

Insbesondere kann die Pulsenergie der Laseranordnung reduziert oder ein Ablationsbereich zu einem späteren Zeitpunkt nochmals angesteuert werden, wenn die erfasste Temperatur des mit dem Licht der Laseranordnung beaufschlagten Auges einen vorbestimmten Grenzwert überschreitet. Dadurch können Schäden an dem zu behandelnden Auge, die durch zu hohe Temperaturen hervorgerufen werden, minimiert oder ganz verhindert werden. Falls erforderlich, kann die Laseranordnung auch vollständig abgeschaltet werden, wenn die erfasste Temperatur des mit dem Licht der Laseranordnung beaufschlagten Auges einen vorbestimmten Grenzwert überschreitet.

Bei einem Verfahren zur Kalibrierung eines Systems zur chirurgischen Behandlung eines Auges wird ein Testobjekt mit Licht beaufschlagt, das von einer Laseranordnung mit einer zur chirurgischen Behandlung des Auges geeigneten Wellenlänge und Repetitionsrate emittiert wird. Das von der Laseranordnung emittierte Licht kann beispielsweise ultraviolettes Licht mit einer Wellenlänge von 193 nm sein. Die Repetitionsrate der Laseranordnung kann von 200 Hz bis 750 Hz betragen, aber auch deutlich höher sein und beispielsweise bei 1050 Hz oder mehr liegen. Ferner wird die Temperatur des mit dem Licht der Laseranordnung zu beaufschlagenden Testobjekts erfasst. Das Testobjekt besteht aus einem Kunststoffmaterial, insbesondere Polymethylmethacrylat (PMMA) bestehen, das sich durch eine hohe Absorption des von der Laseranordnung emittierten Lichts auszeichnet.

Die Temperatur des mit dem Licht der Laseranordnung zu beaufschlagenden Testobjekts kann vor und/oder während der Beaufschlagung des Testobjekts mit dem Licht der Laseranordnung erfasst werden. Mit anderen Worten, bei dem Verfahren zur Kalibrierung eines Systems zur chirurgischen Behandlung eines Auges kann die Ausgangstemperatur des Testobjekts und/oder die Temperatur des Testobjekts während der Beaufschlagung des Testobjekts mit dem Licht der Laseranordnung Berücksichtigung finden. Die Temperatur des mit dem Licht der Laseranordnung zu beaufschlagenden Testobjekts wird berührungslos mittels einer Wärmebildkamera erfasst.

Eine Sollabtragstiefe des Testobjekts kann in Abhängigkeit von einer Ausgangstemperatur des mit dem Licht der Laseranordnung zu beaufschlagenden Testobjekts angepasst werden.

Ein Parametersatz für ein Überwachungsprogramm zur Überwachung der Laseranordnung während der chirurgischen Behandlung eines Auges wird vorzugsweise in Abhängigkeit eines von der Temperaturerfassungseinrichtung ausgegebenen Temperatursignals erstellt. Beispielsweise kann der Parametersatz für das Überwachungsprogramm in Abhängigkeit eines von der Temperaturerfassungseinrichtung ausgegebenen, die Ausgangstemperatur des Testobjekts charakterisierenden Temperatursignals erstellt werden. Zusätzlich oder alternativ dazu kann bei der Erstellung des Parametersatzes für das Überwachungsprogramm ein von der Temperaturerfassungseinrichtung ausgegebenes, die Temperatur des Testobjekts während der Beaufschlagung mit dem Licht der Laseranordnung charakterisierendes Temperatursignal berücksichtigt werden.

Insbesondere wird in dem Überwachungsprogramm zur Überwachung der Laseranordnung während der chirurgischen Behandlung eines Auges eine zur Erzielung eines gewünschten Behandlungsergebnisses erforderliche Pulsenergie der Laseranordnung in Abhängigkeit des von der Temperaturerfassungseinrichtung ausgegebenen Temperatursignals bestimmt. Beispielsweise kann in dem Überwachungsprogramm hinterlegt sein, welche Pulsenergie der Laseranordnung erforderlich ist, um eine gewünschte Abtragstiefe, d. h. eine gewünschte Dicke der durch das Licht der Laseranordnung abgetragenen Gewebeschicht zu erzielen. Hierzu kann im Rahmen des Verfahrens zur Kalibrierung des Behandlungssystems eine Korrelation der Leistung der Laseranordnung mit der erzielten Abtragstiefe in Abhängigkeit der Temperatur des Testobjekts bestimmt und anschließend eine Korrektur dieser Daten unter Berücksichtigung der Materialeigenschaften des Testobjekts und des zu behandelnden Teils eines Auges, beispielsweise der Kornea des Auges, durchgeführt werden. Die Erfindung ist durch die Ansprüche definiert. Ein bevorzugtes Ausführungsbeispiel der Erfindung wird nun anhand der beigefügten, schematischen Zeichnungen näher erläutert, von denen
- Figur 1: eine schematische Darstellung eines Systems zur chirurgischen Behandlung eines Auges, das nicht Teil der Erfindung ist, zeigt, und
- Figur 2: ein Flussdiagramm zeigt, das die Schritte eines Verfahrens zur Kalibrierung eines Systems zur chirurgischen Behandlung eines Auges sowie eines nicht erfindungsgemäßen Verfahrens zur chirurgischen Behandlung eines Auges veranschaulicht.

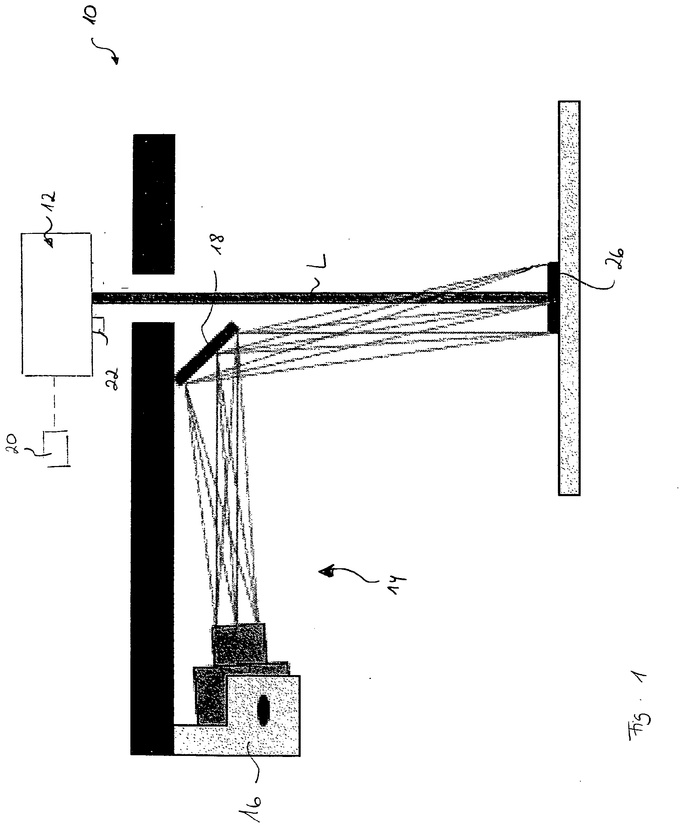

Ein in Figur 1 veranschaulichtes System 10 zur chirurgischen Behandlung eines Auges umfasst eine Laseranordnung 12 mit einem Argonfluorid-Excimerlaser, die ultraviolettes Licht mit einer Wellenlänge von 193 nm emittiert. Die Laseranordnung 12 arbeitet mit einer Repetitionsrate von 1050 Hz. In dem System 10 kann jedoch auch eine Laseranordnung mit einer geringeren Repetitionsrate von beispielsweise 200 Hz bis 750 Hz zum Einsatz kommen.

Ferner ist das System 10 mit einer Temperaturerfassungseinrichtung 14 ausgestattet. Die Temperaturerfassungseinrichtung 14 umfasst eine Wärmebildkamera 16, die anhand ihr zugeführter IR-Strahlung berührungslos Temperaturmessungen vornehmen kann. Ferner umfasst die Temperaturerfassungseinrichtung 14 eine Umlenkeinrichtung 18 zur Umlenkung der der Wärmebildkamera 16 zugeleiteten IR-Strahlung. In dem in Figur 1 gezeigten Beispiel eines Systems 10 zur chirurgischen Behandlung eines Auges besteht die Umlenkeinrichtung 18 aus einem mit Silber, Aluminium oder Gold beschichteten Spiegel, der im Strahlengang der der Wärmebildkamera 16 zugeleiteten IR-Strahlung angeordnet ist.

Die Laseranordnung 12 umfasst eine Steuereinrichtung 20, die dazu dient, den Betrieb der Laseranordnung 12 und insbesondere die Pulsenergie der Laseranordnung 12 zu steuern. Darüber hinaus steuert die Steuereinrichtung 20 einen mit zwei Ablenkachsen ausgestatteten Scanner 22. Der Scanner 22 dient dazu, das von der Laseranordnung 12 emittierte Licht, d. h. den Laserstrahl L, über eine Oberfläche eines mit dem Licht der Laseranordnung 12, d. h. dem Laserstrahl L zu beaufschlagenden Objekts 26 zu bewegen.

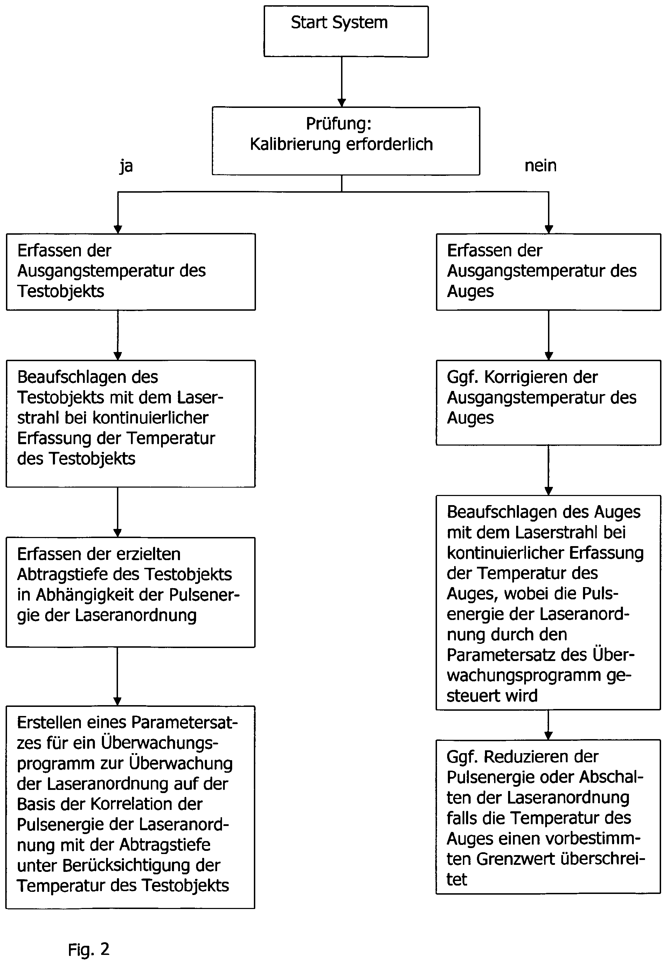

Im Folgenden wird die Kalibrierung sowie die Funktion des in Figur 1 veranschaulichten Systems 10 zur chirurgischen Behandlung eines Auges erläutert. Wie in Figur 2 veranschaulicht, wird nach dem Start des Systems 10 zunächst geprüft, ob eine Kalibrierung des Systems 10 erforderlich ist. Beispielsweise kann eine Kalibrierung des Systems 10 beim ersten Einschalten des Systems 10 für erforderlich erachtet werden oder nach einer bestimmten Anzahl von mittels des Systems 10 durchgeführten Behandlungen oder nach einer vorbestimmten Anzahl von Einsatzstunden des Systems 10. Darüber hinaus kann eine von dem System 10 ausgegebene Störungsmeldung eine Kalibrierung des Systems erforderlich machen. Schließlich ist es auch denkbar, dass ein Bediener des Systems 10 entscheiden kann, eine Kalibrierung des Systems 10 durchzuführen.

Wenn in dem oben beschriebenen Prüfschritt festgestellt wird, dass eine Kalibrierung des Systems 10 erforderlich ist, wird ein im Folgenden beschriebenes Kalibrierungsverfahren durchgeführt. Im Rahmen dieses Kalibrierungsverfahrens wird zunächst mittels der Wärmebildkamera 16 eine Ausgangstemperatur eines Testobjekts erfasst. Als Testobjekt kann beispielsweise ein aus einem Kunststoffmaterial, insbesondere aus Polymethylmethacrylat bestehendes Testobjekt Anwendung finden. Anschließend wird das Testobjekt mit dem von der Laseranordnung 12 emittierten Laserstrahl L beaufschlagt. Während der Beaufschlagung des Testobjekts mit dem Laserstrahl L wird mittels der Wärmebildkamera 16 kontinuierlich die Temperatur des Testobjekts erfasst.

Bei der Erfassung der Ausgangstemperatur des Testobjekts erfolgt die Temperaturmessung, ebenso wie bei der kontinuierlichen Erfassung der Temperatur des Testobjekts während der Beaufschlagung des Testobjekts mit dem Laserstrahl L, über eine definierte Fläche des Testobjekts, die eine Größe von z.B. 20x20 mm aufweist. Für jede Temperaturmessung werden drei Messungen in einem Abstand von z.B. 5 Sekunden über eine Dauer von z.B. 0,5 Sekunden durchgeführt und die Messwerte gemittelt. Der Mittelwert aus diesen drei Messungen wird dann jeweils als Temperaturwert des Testobjekts angesehen.

Ferner wird die erzielte Abtragstiefe des Testobjekts in Abhängigkeit der Pulsenergie der Laseranordnung 12 erfasst und diese Werte mit den gemessenen Temperaturwerten des Testobjekts korreliert. Schließlich wird die Kalibrierungsmessung zur Erstellung eines Parametersatzes für ein Überwachungsprogramms zur Überwachung der Laseranordnung 12 auf der Basis der Korrelation der Pulsenergie der Laseranordnung 12 mit der Abtragstiefe des Testobjekts unter Berücksichtigung der Temperatur des Testobjekts genutzt. Mit anderen Worten, es wird ein Parametersatz erstellt, das es ermöglicht, die Laseranordnung 12, d. h. insbesondere die Pulsenergie der Laseranordnung 12 so zu steuern, dass eine gewünschte Abtragstiefe erzielt, gleichzeitig eine gewünschte Maximaltemperatur eines zu behandelnden Auges jedoch nicht überschritten wird. Es versteht sich, dass bei der Erstellung der Parameter die im Rahmen der Kalibrierungsmessung für das Testobjekt erfassten Daten unter Berücksichtigung der Materialeigenschaften des Testobjekts und des zu behandelnden Teils eines Auges, beispielsweise der Kornea des Auges, korrigiert werden müssen. Ergebnis der Kalibrierung des Systems 10 ist folglich ein Parametersatz, der es ermöglicht, die zur Erzielung einer gewünschten Abtragstiefe erforderliche Pulsenergie der Laseranordnung 12 bereits unter Berücksichtigung der Auswirkungen des Laserstrahls L auf die Temperatur eines mit dem Laserstrahl L zu behandelnden Auges zu steuern.

Bei der Durchführung eines in Figur 2 auf der rechten Seite veranschaulichten Verfahrens zur chirurgischen Behandlung eines Auges, das nicht Teil der Erfindung ist, wird zunächst die Ausgangstemperatur des zu behandelnden Auges, d. h. insbesondere die Ausgangstemperatur der Kornea des Auges, erfasst. Diese Temperaturmessung erfolgt mittels der Wärmebildkamera 16. Falls gewünscht oder erforderlich, kann die Ausgangstemperatur des Auges angepasst, beispielsweise durch Kühlen des Auges bzw. der Kornea des Auges reduziert werden.

Anschließend wird das Auge mit dem Laserstrahl L beaufschlagt, wobei während der Beaufschlagung des Auges mit dem von der Laseranordnung 12 emittierten Laserstrahl L die Temperatur des Auges mittels der Wärmebildkamera 16 kontinuierlich erfasst wird. Die Pulsenergie der Laseranordnung 12 wird dabei durch den im Rahmen des oben beschriebenen Kalibrierungsverfahrens erstellten Parametersatz gesteuert. Darüber hinaus wird von der Steuereinrichtung 20 die Pulsenergie der Laseranordnung 12 reduziert bzw. ein Ablationsbereich zu einem späteren Zeitpunkt nochmals angesteuert, falls die Temperatur des Auges einen vorbestimmten Grenzwert überschreitet. Auf diese Weise werden Temperaturschäden des Auges zuverlässig vermieden.

## Patentansprüche

1. Verfahren zur Kalibrierung eines Systems zur chirurgischen Behandlung eines Auges mit einer Laseranordnung (12), die dazu eingerichtet ist, Licht mit einer zur chirurgischen Behandlung des Auges geeigneten Wellenlänge und Repetitionsrate zu emittieren,
mit einer Temperaturerfassungseinrichtung (14), die dazu eingerichtet ist, die Temperatur eines mit dem Licht der Laseranordnung (12) zu beaufschlagenden Objekts (26) zu erfassen, und
einer Steuereinrichtung (20), die dazu eingerichtet ist, ein von der Temperaturerfassungseinrichtung (14) ausgegebenes Temperatursignal zu empfangen und die Laseranordnung (12) in Abhängigkeit dieses Temperatursignals zu steuern, wobei die Steuereinrichtung (20) dazu eingerichtet ist, wenn die von der Temperaturerfassungseinrichtung (14) erfasste Temperatur des mit dem Licht der Laseranordnung (12) beaufschlagten Objekts (26) einen vorbestimmten Grenzwert überschreitet, einen Ablationsbereich zu einem späteren Zeitpunkt nochmals derart anzusteuern, dass durch eine unerwünschte Temperaturerhöhung verursachte Schäden an einem zu behandelnden Auge vermieden werden, wobei das Verfahren umfasst:
- Beaufschlagen eines Testobjekts mit Licht, das von der Laseranordnung (12) mit der zur chirurgischen Behandlung des Auges geeigneten Wellenlänge und Repetitionsrate emittiert wird,
wobei die Temperatur des mit dem Licht der Laseranordnung (12) zu beaufschlagenden Testobjekts erfasst wird, und
wobei das Testobjekt aus einem Kunststoffmaterial besteht, und wobei die Temperatur des mit dem Licht der Laseranordnung (12) zu beaufschlagenden Testobjekts vor und/oder während der Beaufschlagung des Testobjekts mit dem Licht der Laseranordnung (12) erfasst wird, und wobei die Temperatur des mit dem Licht der Laseranordnung (12) zu beaufschlagenden Testobjekts mittels einer Wärmebildkamera (16) erfasst wird.

2. Verfahren nach einem der Ansprüche 1, wobei eine Sollabtragstiefe des Testobjekts in Abhängigkeit von einer Ausgangstemperatur des mit dem Licht der Laseranordnung (12) zu beaufschlagenden Testobjekts angepasst wird.

3. Verfahren nach einem der Ansprüche 1 bis 2, wobei ein Parametersatz für ein Überwachungsprogramm zur Überwachung der Laseranordnung (12) während der chirurgischen Behandlung eines Auges in Abhängigkeit eines von der Temperaturerfassungseinrichtung (14) ausgegebenen Temperatursignals erstellt wird.

4. Verfahren nach Anspruch 3, wobei in dem Parametersatz eine zur Erzielung eines gewünschten Behandlungsergebnisses erforderliche Pulsenergie der Laseranordnung (12) in Abhängigkeit des von der Temperaturerfassungseinrichtung (14) ausgegebenen Temperatursignals bestimmt wird.

## Claims

1. Method for calibrating a system for surgical treatment of an eye using a laser arrangement (12) which is configured to emit light with a wavelength and repetition rate that are suitable for the surgical treatment of the eye,
comprising a temperature capture device (14) which is configured to capture the temperature of an object (26) to be struck by the light from the laser arrangement (12), and
a control device (20) which is configured to receive a temperature signal output by the temperature capture device (14) and control the laser arrangement (12) dependent on this temperature signal, wherein the control device (20) is configured to drive again to an ablation region at a subsequent time in such a way that damage, caused by an unwanted temperature increase, to an eye to be treated is avoided if the temperature, captured by the temperature capture device (14), of the object (26) struck by the light from the laser arrangement (12) exceeds a predetermined threshold, wherein the method comprises:
- striking the test object with the light which is emitted by the laser arrangement (12) with the wavelength and repetition rate that are suitable for the surgical treatment of the eye,
wherein the temperature of the test object to be struck by the light from the laser arrangement (12) is captured, and
wherein the test object consists of a plastics material, and
wherein the temperature of the test object to be struck by the light from the laser arrangement (12) is captured before and/or while the test object is struck by the light from the laser arrangement (12), and
wherein the temperature of the test object to be struck by the light from the laser arrangement (12) is captured by means of the thermal imaging camera (16).

2. Method according to Claim 1,
wherein an intended ablation depth of the test object is adapted depending on an initial temperature of the test object which is to be struck by the light from the laser arrangement (12).

3. Method according to either of Claims 1 and 2,
wherein a parameter set for a monitoring program for monitoring the laser arrangement (12) during the surgical treatment of an eye is created depending on a temperature signal output by the temperature capture device (14).

4. Method according to Claim 3,
wherein a pulse energy of the laser arrangement (12) that is required to obtain a desired treatment result is determined in the parameter set depending on the temperature signal output by the temperature capture device (14).

## Revendications

1. Procédé d'étalonnage d'un système de traitement chirurgical de l'oeil comprenant un ensemble laser (12) conçu pour émettre de la lumière ayant une longueur d'onde et une fréquence de répétition appropriées au traitement chirurgical de l'oeil,
comprenant un dispositif de détection de température (14) conçu pour détecter la température d'un objet (26) à exposer à la lumière de l'ensemble laser (12), et un dispositif de commande (20) conçu pour recevoir un signal de température délivré par le dispositif de détection de température (14) et pour commander l'ensemble laser (12) en fonction dudit signal de température, dans lequel le dispositif de commande (20) est conçu pour commander à nouveau à un instant ultérieur une zone d'ablation de telle sorte que l'endommagement d'un oeil à traiter provoqué par une augmentation indésirable de la température soit évité si la température de l'objet (26) exposé à la lumière de l'ensemble laser (12), détectée par le dispositif de détection de température (14), dépasse une valeur limite prédéterminée, dans lequel le procédé consiste à :
- exposer un objet de test à de la lumière émise par ledit ensemble laser (12) et ayant la longueur d'onde et la fréquence de répétition appropriées pour le traitement chirurgical de l'oeil,
dans lequel la température de l'objet de test à exposer à la lumière de l'ensemble laser (12) est détectée, et dans lequel l'objet de test est constitué d'une matière plastique, et
dans lequel la température de l'objet de test à exposer à la lumière de l'ensemble laser (12) est détectée avant et/ou pendant l'exposition de l'objet de test à la lumière de l'ensemble laser (12), et
dans lequel la température de l'objet de test à exposer à la lumière de l'ensemble laser (12) est détectée au moyen d'une caméra thermique (16).

2. Procédé selon la revendication 1,
dans lequel une profondeur nominale de retrait de l'objet de test est adaptée en fonction d'une température initiale de l'objet de test à exposer à la lumière de l'ensemble laser (12).

3. Procédé selon l'une des revendications 1 à 2, dans lequel un jeu de paramètres utilisés dans un programme de surveillance destiné à surveiller l'ensemble laser (12) pendant le traitement chirurgical d'un oeil est établi en fonction d'un signal de température délivré par le dispositif de détection de température (14).

4. Procédé selon la revendication 3,
dans lequel une énergie d'impulsion de l'ensemble laser (12) nécessaire pour obtenir un résultat de traitement souhaité est déterminée dans le jeu de paramètres en fonction du signal de température délivré par le dispositif de détection de température (14).
